(19) Europäisches Patentamt
European Patent Office
Office européen des brevets

(11) Publication number: **0 267 629**
**A2**

(12) **EUROPEAN PATENT APPLICATION**

(21) Application number: **87116824.1**

(22) Date of filing: **13.11.87**

(51) Int. Cl.⁴: **C07K 7/06** , C07K 7/08 , C07K 7/10 , C12N 1/20 , C12N 15/00 , C12P 21/06 , C07K 1/00 , //C12P21/02,A61K37/02,(C12P-21/06,C12R1:025,1:445),(C12N-1/20,C12R1:19)

(30) Priority: **14.11.86 JP 271605/86**

(43) Date of publication of application: **18.05.88 Bulletin 88/20**

(84) Designated Contracting States: **DE FR GB**

(71) Applicant: **KYOWA HAKKO KOGYO CO., LTD.**
**6-1, Ohte-Machi 1-chome**
**Chiyoda-ku Tokyo-to(JP)**

(72) Inventor: **Matsumoto, Tadashi**
**3549-3, Honmachida**
**Machida-shi Tokyo(JP)**
Inventor: **Harada, Nahoko**
**904-11, Honmachida**
**Machida-shi Tokyo(JP)**
Inventor: **Yamaguchi, Kazuo**
**2121-8, Isobe**
**Sagamihara-shi Kanagawa(JP)**
Inventor: **Komatsu, Yoshinori**
**2662-13, Honmachida**
**Machida-shi Tokyo(JP)**
Inventor: **Itoh, Seiga**
**218-14, Aihara Aza Hachimannishi**
**Sagamihara-shi Kanagawa(JP)**

(74) Representative: **Kinzebach, Werner, Dr.**
**Patentanwälte Reitstötter, Kinzebach & Partner Sternwartstrasse 4 Postfach 86 06 49**
**D-8000 München 86(DE)**

(54) **Polypeptides having interleukin-1 activity.**

(57) Novel polypeptides having interleukin 1 (IL-1) activity and a method of producing the polypeptides which comprises producing human IL-1 by using gene engineering technique and treating the human IL-1 with an enzyme.

EP 0 267 629 A2

# POLYPEPTIDES HAVING INTERLEUKIN-1 ACTIVITY

## FIELD OF THE INVENTION

This invention relates to novel polypeptides having interleukin-1 activity, DNA fragments coding for said polypeptides, recombinant plasmids with any of said DNA fragments inserted therein, microorganisms harboring said plasmids, and a method of producing polypeptides having interleukin-1 activity in which said microorganisms are used.

## BACKGROUND OF THE INVENTION

Interleukin-1 (hereinafter abbreviated as IL-1) is one of the monokines produced by activated macrophages. IL-1 acts on T cells, among others, and shows immuno-potentiating activity. Therefore, IL-1 is expected to be clinically useful as an anticancer or antiinfective agent. The polypeptides of this invention which show IL-1 activity are expected to be of use as anticancer or antiinfective agents, like IL-1.

Recent rapid advances in recombinant DNA technology have made it possible to produce, in microorganisms, large quantities of those substances which are produced only in small quantities in higher animal cells and therefore are difficult to recover.

As regards IL-1, the cloning of a human peripheral blood macrophage-derived cDNA in Escherichia coli and the determination of its base sequence have been reported by Auron et al. [Philip E. Auron et al.. Proc. Natl. Acad. Sci. USA, 81 , 7907 (1984)]. Also, short peptide fragments of human IL-1 have been reported by Rosenwasser et al. and Antoni et al. [Larry J. Rosenwasser et al., Proc. Natl. Acad. Sci. USA, 83, 5243-5246 (1986); G. Antoni et al., J. Immunol, 137, 3201-3204 (1986)].

## SUMMARY OF THE INVENTION

It is an object of this invention to provide a means of producing, at low cost and in large quantities, polypeptides having IL-1 activity and substantially no pyrogenicity in mammals.

The present inventors cloned a human peripheral blood macrophage-derived cDNA in Escherichia coli and determined the whole base sequence thereof, which is shown below in Table 1. The amino acid sequence encoded by this base sequence is in agreement with that reported by Auron et al.

The inventors then constructed an IL-1 expression plasmid based on the cDNA for IL-1 as shown in Table 1, cultured a strain of Escherichia coli with the cDNA cloned therein, highly purified the thus-produced IL-1, and found that polypeptides showing IL-1 activity can be produced by degrading said IL-1 enzymatically. This finding has now led to completion of the present invention.

Table 1

```
                                 ACAAACCTTTTCGAGGCAAAAGGCAA

AAAAGGCTGCTCTGGGATTCTCTTCAGCCAATCTTCAATGCTCAAGTGTCTGAAGCAGCC

        10        20        30        40        50        60
ATGGCAGAAGTACCTAAGCTCGCCAGTGAAATGATGGCTTATTACAGTGGCAATGAGGAT
MetAlaGluValProLysLeuAlaSerGluMetMetAlaTyrTyrSerGlyAsnGluAsp

        70        80        90       100       110       120
GACTTGTTCTTTGAAGCTGATGGCCCTAAACAGATGAAGTGCTCCTTCCAGGACCTGGAC
AspLeuPhePheGluAlaAspGlyProLysGlnMetLysCysSerPheGlnAspLeuAsp

       130       140       150       160       170       180
CTCTGCCCTCTGGATGGCGGCATCCAGCTACGAATCTCCGACCACCACTACAGCAAGGGC
LeuCysProLeuAspGlyGlyIleGlnLeuArgIleSerAspHisHisTyrSerLysGly

       190       200       210       220       230       240
TTCAGGCAGGCCGCGTCAGTTGTTGTGGCCATGGACAAGCTGAGGAAGATGCTGGTTCCC
PheArgGlnAlaAlaSerValValValAlaMetAspLysLeuArgLysMetLeuValPro

       250       260       270       280       290       300
TGCCCACAGACCTTCCAGGAGAATGACCTGAGCACCTTCTTTCCCTTCATCTTTGAAGAA
CysProGlnThrPheGlnGluAsnAspLeuSerThrPhePheProPheIlePheGluGlu

       310       320       330       340       350       360
GAACCTATCTTCTTCGACACATGGGATAACGAGGCTTATGTGCACGATGCACCAGTACGA
GluProIlePhePheAspThrTrpAspAsnGluAlaTyrValHisAspAlaProValArg

       370       380       390       400       410       420
TCACTGAACTGCACGCTCCGGGACTCACAGCAAAAAAGCTTGGTGATGTCTGGTCCATAT
SerLeuAsnCysTyrLeuArgAspSerGlnGlnLysSerLeuValMetSerGlyProTyr
```

```
        430       440       450       460       470       480
GAACTGAAAGCTCTCCACCTCCAGGGACAGGATATGGAGCAACAAGTGGTGTTCTCCATG
GluLeuLysAlaLeuHisLeuGlnGlyGlnAspMetGluGlnGlnValValPheSerMet

        490       500       510       520       530       540
TCCTTTGTACAAGGAGAAGAAAGTAATGACAAAATACCTGTGGCCTTGGGCCTCAAGGAA
SerPheValGlnGlyGluGluSerAsnAspLysIleProValAlaLeuGlyLeuLysGlu

        550       560       570       580       590       600
AAGAATCTGTACCTGTCCTGCGTGTTGAAAGATGATAAGCCCACTCTACAGCTGGAGACT
LysAsnLeuTyrLeuSerCysValLeuLysAspAspLysProThrLeuGlnLeuGluThr

        610       620       630       640       650       660
GTACATCCCAAAAATTACCCAAAGAAGAAGATGGAAAAGCGATTTGTCTTCAACAAGATA
ValHisProLysAsnTyrProLysLysLysMetGluLysArgPheValPheAsnLysIle

        670       680       690       700       710       720
GAAATCAATAACAAGCTGGAATTTGAGTCTGCCCAGTTCCCCAACTGGTACATCAGCACC
GluIleAsnAsnLysLeuGluPheGluSerAlaGlnPheProAsnTrpTyrIleSerThr

        730       740       750       760       770       780
TCTCAAGCAGAAAACATGCCCGTCTTCCTGGGAGGGACCAAAGGCGGCCAGGATATAACT
SerGlnAlaGluAsnMetProValPheLeuGlyGlyThrLysGlyGlyGlnAspIleThr

        790       800       810
GACTTCACCATGCAATTTGTGTCTTCCTAAAGAGAGCTGTACCCAGAGAGTCCTGTGCTG
AspPheThrMetGlnPheValSerSer***

AATGTGGACTCAATCCCTAGGGCTGGCAGAAAGGGAACAGAAAGGTTTTTGAGTACGGCT
```

The invention thus provides novel polypeptides showing IL-1 activity. DNA fragments coding for these polypeptides, recombinant plasmids with the DNA fragments inserted therein, microorganisms harboring the plasmids, and a method of producing such novel polypeptides with IL-1 activity which comprises the use of the microorganisms.

## BRIEF DESCRIPTION OF THE DRAWINGS

In the accompanying drawings,

Fig. 1 shows a construction scheme for the recombinant plasmid pLIC12 discussed below;

Fig. 2 shows an outlined process for cDNA synthesis by the Okayama-Berg method and for the construction of a recombinant plasmid containing the thus-synthesized DNA; and

Fig. 3 shows a construction scheme for the ATG vector pTrS20 also discussed below.

## DETAILED DESCRIPTION OF THE INVENTION

The polypeptides of the invention are polypeptides obtained by treating interleukin-1 with an enzyme, such as Achromobacter protease I or Staphylococcus aureus V8 protease, and are characterized by their interleukin-1 activity and being substantially free of pyrogenicity in mammals. Specific examples of the polypeptides according to the invention have the following amino acid sequences:

(a) Arg-Phe-Val-Phe-Asn-Lys (Formula A-4),

(b) Lys-Arg-Phe-Val-Phe-Asn-Lys-Ile-Glu (Formula V8-7),

(c) Ser-Leu-Asn-Cys-Thr-Leu-Arg-Asp-Ser-Gln-Gln-Lys (Formula A-3),

(d) Asn-Tyr-Pro-Lys-Lys-Lys-Met-Glu-Lys-Arg-Phe-Val-Phe-Asn-Lys-Ile-Glu-Ile-Asn-Asn-Lys (Formula A-10), and

(e) Leu-Lys-Ala-Leu-His-Leu-Gln-Gly-Gln-Asp-Met-Glu-Gln-Gln-Val-Val-Phe-Ser-Met-Ser-Phe-Val-Gln-Gly-Glu-Glu-Ser-Asn-Asp-Lys-Ile-Pro-Val-Ala-Leu-Gly-Leu-Lys-Glu-Lys-Asn-Leu-Tyr-Leu-Ser-Cys-Val-Leu-Lys-Asp-Asp-Lys-Pro-Thr-Leu-Gln-Leu-Glu (Formula V8-11).

The symbols of amino acid residues as used in this specification and in the appended claims are as follows: Ala, alanine; Arg, arginine; Asn, asparagine; Asp, aspartic acid; Cys, cysteine; Gln, glutamine; Glu, glutamic acid; Gly, glycine; His, histidine; Ile, isoleucine; Leu, leucine; Lys, lysine; Met, methionine; Phe, phenylalanine; Pro, proline; Ser, serine; Thr, threonine; Tyr, tyrosine; and, Val, valine. The polypeptides of the above formulae are hereinafter referred to in this specification and in the appended claims as A-4, V8-7, A-3, A-10 and V8-11, respectively.

Human interleukin-1 can be obtained by cloning the human peripheral blood macrophage-derived IL-1 cDNA in Escherichia coli, cultivating the resultant Escherichia coli transformant in a medium to thereby cause accumulation of IL-1 in the culture, and recovering the IL-1 from the culture. The plasmid pcDIL-1 or pCD-415 may be used as a source of said IL-1 cDNA. pcDIL-1 can be produced as described later in Reference Example 1. pCD-415 can be produced as described by Auron et al in Proc. Natl. Acad. Sci. USA, 81, 7907 (1984), noted above.

Any plasmid may be used for the insertion therein of the human IL-1-encoding DNA provided that the DNA can be expressed in Escherichia coli. Preferably a plasmid is used which has the initiation codon (ATG) downstream from an appropriate promoter, such as a trp or lac promoter, retains the initiation codon on the 3′ end side upon cleavage with an appropriate restriction enzyme, allows foreign DNA insertion and has the distance between the Shine-Dalgarno sequence (hereinafter abbreviated as SD sequence) and the initiation codon adjusted to an appropriate distance, for example, 6-18 base pairs. A suitable example of such plasmid (ATG vector) is pTrS20 (Reference Example 2).

A recombinant plasmid, pLIC12, containing the human IL-1-encoding DNA inserted therein can be constructed, for example, by using pcDIL-1 as the human IL-1 cDNA source and pTrS20 as a plasmid for providing said cDNA with the initiation codon, as follows:

As shown in Fig. 1, pcDIL-1 is cleaved with BamHI and Dpn I, and a DNA fragment of about 1,000 base pairs (hereinafter abbreviated as bp) is purified by low gelling temperature agarose gel electrophoresis (LTG method) [L. Wieslander, Analytical Biochemistry, 98, 305 (1979)]. Separately, pTrS20 is cleaved with SacI, followed by treatment with T4 polymerase and cleavage with PstI to give a 1.1 kb DNA fragment. Furthermore, pGELI is cleaved with Pst I and BamHI to give a 1.7 kb DNA fragment. The thus-obtained DNA fragments are joined together using T4 DNA ligase, whereby pLIC12 is obtained.

The reaction conditions of the above recombination procedure are generally as follows:

The DNA digestion reactions in the presence of a restriction enzyme or enzymes are generally carried out using 0.1-20 µg of DNA in a reaction medium containing 2-200 mM (preferably 10-40 mM) Tris-HCl (pH 6.0-9.5, preferably 7.0-8.0), 0-200 mM NaCl and 2-20 mM (preferably 5-10 mM) $MgCl_2$ at 20-70°C (the optimal temperature varying depending on the restriction enzyme(s) used) for 15 minutes to 24 hours. The restriction enzymes are each used in an amount of 0.1-100 units (preferably 1-3 units) per microgram of DNA. Termination of the reactions is generally effected by heating at 55-75°C for 5-30 minutes. It is also possible to inactivate the restriction enzymes with a reagent such as phenol or diethyl pyrocarbonate.

The DNA fragments formed from the restriction enzyme digestion are purified by the above-mentioned LGT method, for instance.

DNA fragment ligation is carried out in a reaction medium containing 2-200 mM (preferably 10-40 mM) Tris-HCl (pH 6.1-9.5, preferably 7.0-8.0), 2-20 mM (preferably 5-10 mM) $MgCl_2$, 0.1-10 mM (preferably 0.5-2.0 mM) ATP and 1-50 mM (preferably 5-10 mM) dithiothreitol at 1-37°C (preferably 3-20°C) for 15 minutes to 72 hours (preferably 2-20 hours), using 0.3-10 units of T4 DNA ligase.

The recombinant plasmid DNAs formed by the ligation reactions are introduced into Escherichia coli by the transformation method of Cohen et al. [S.N. Cohen et al., Proc. Natl. Acad. Sci. USA, 69, 2110 (1972)], as necessary.

Isolation of the recombinant plasmid DNAs from strains of Escherichia coli harboring them is performed by the method of Birnboim et al. [H.C. Birnboim et al., Nucleic Acids Res., 7, 1513 (1979)], for instance.

The plasmid DNAs are examined for cleavage sites by agarose gel electrophoresis or polyacrylamide gel electrophoresis following cleavage with 1-10 restriction enzymes. Further DNA base sequence determination is performed, if necessary, by the method of Messing [Gene, 19, 269 (1982)].

Under conditions such as mentioned above, the de sired recombinant plasmid DNAs can be produced.

The polypeptides of the invention which show IL-1 activity can be produced in the following manner. A suitable plasmid (e.g., pLIC12) is used to transform Escherichia coli KM430 (deposited at the Fermentation Research Institute, Agency of Industrial Science and Technology as of February 1, 1986 under the deposit number FERM BP-981 in accordance with the Budapest treaty), and a pLIC12-bearing transformant of Escherichia coli is selected from among ampicillin-resistant (hereinafter, $Ap^r$) colonies. Growing the pLIC12-bearing strain of Escherichia coli in a medium leads to formation of a precursor polypeptide to the desired polypeptides in the medium.

Any medium, whether synthetic or natural, may be used provided that it is suited for the growth of Escherichia coli and for the production of the above-mentioned precursor polypeptide.

Usable carbon sources include glucose, fructose, lactose, glycerol, mannitol and sorbitol, among others, and usable nitrogen sources are $NH_4Cl$, $(NH_4)_2SO_4$, casamino acids, yeast extract, polypeptone, meat extract, Bactotryptone, corn steep liquor, etc. $K_2HPO_4$, $KH_2PO_4$, NaCl, $MgSO_4$, vitamin $B_1$, $MgCl_2$ and so forth may be used as other nutrient sources.

The cultivation is carried out with aeration and stirring at a pH of 5.5-8.5 and a temperature of 18-40°C. Cultivation for 5-90 hours leads to accumulation of IL-1 in cultured cells. The cells are then harvested from the culture and disrupted by sonication. Following centrifugation, IL-1 can be separated and purified from the supernatant by precipitation with 65% ammonium sulfate. Further extreme purification of IL-1 can be realized by high-performance liquid chromatography (HPLC). Protein quantity determination can be made by the method of M.M. Bradford [Analytical Biochemistry, 72 , 248 (1976)]. The content of IL-1 in samples can be determined by using a chromato scanner following SDS-polyaccrylamide gel electrophoresis by the method of Laemmli [U.K. Laemmli, Nature, 227, 680 (1970)]. Treatment of the thus-obtained IL-1 with an enzyme [Achromobacterprotease I (hereinafter abbreviated as API) or Staphylococcus aureus V8 protease (hereinafter abbreviated as V8 protease)] under appropriate conditions gives the polypeptides of the invention which show IL-1 activity.

The enzymatic degradation of IL-1 and the recovery of degradation product polypeptides are conducted in the following manner, for instance.

For API degradation, 100 µg of IL-1 and 2 µg of the enzyme are dissolved in 0.01 M Tris-4 M urea (pH 9.1) and the reaction is conducted at 37°C for 5 hours.

For V8 protease degradation, 100 µg of IL-1 and 2.5 µg of the enzyme are dissolved in 0.1 M Tris (pH 7.8) and the reaction is conducted at 37°C for 22 hours.

After reaction, fragments are fractionated on a Yamamura Kagaku model YMC AM312 ODS reversed-phase column.

The polypeptides according to the invention can be synthesized by using an automatic peptide synthesizer with reagents and solvents suited therefor while following a standard operational program

adapted to the synthesizer employed. An example of synthesis of V-4, one of the polypeptides according to the invention, is given in Example 7. Other polypeptides can be synthesized in the same manner.

The following examples illustrate the invention in further detail.

## EXAMPLE 1

### Construction of plasmid pLIC12

A 10-$\mu$g portion of pcDIL-1 (4750 bp) obtained as described in Reference Example 1 was dissolved in 50 $\mu$l of a solution (hereinafter referred to as Y-100 buffer) containing 10 mM Tris-HCl (pH 7.5), 7 mM $MgCl_2$, 100 mM NaCl and 6 mM 2-mercaptoethanol, 20 units of the restriction enzyme BamHI (Takara Shuzo; hereinafter, unless otherwise specified, all the restriction enzymes used were products of Takara Shuzo) was added, and the cleavage reaction was carried out at 37°C for 2 hours. Then, the cleaved DNA was extracted with phenol-chloroform, precipitated with ethanol and dissolved in 50 $\mu$l of a solution (hereinafter referred to as Y-150 buffer) containing 10 mM Tris-HCl (pH 7.5), 7 mM $MgCl_2$, 150 mM NaCl and 6 mM 2-mercaptoethanol and, after addition of 10 units of the restriction enzyme DpnI, the cleavage reaction was conducted at 37°C for 30 minutes (partial digestion). About 0.5 $\mu$g of a human IL-1 DNA-containing DNA fragment (DpnI-BamHI fragment) of about 1,000 bp was recovered from the reaction mixture by the LGT method.

Separately, 10 $\mu$g of pTrS20 (Reference Example 2) was dissolved in 50 $\mu$l of a solution (hereinafter referred to as Y-0 buffer) containing 10 mM Tris-HCl (pH 7.5), 7 mM $MgCl_2$ and 6 mM 2-mercaptoethanol, 20 units of the restriction enzyme SacI was added, and the cleavage reaction was conducted at 37°C for 2 hours. Then, the cleaved DNA was extracted with phenol-chloroform, precipitated with ethanol and dissolved in 50 $\mu$l of a solution containing 67 mM Tris-HCl (pH 8.8), 6.7 mM $MgCl_2$, 10 mM 2-mercaptoethanol, 16.7 mM $(NH_4)_2SO_4$, 6.7 mM EDTA, 1 mM dATP, 1 mM dGTP, 1 mM dCTP and 1 mM dTTP and, following addition of 4 units of T4 DNA polymerase, the reaction was conducted at 37°C for 1 hour. The resultant DNA was extracted with phenol-chloroform, precipitated with ethanol and dissolved in 50 $\mu$l of a solution (hereinafter referred to as Y-50 buffer) containing 10 mM Tris-HCl (pH 7.5), 7 mM $MgCl_2$, 50 mM NaCl and 6 mM 2-mercaptoethanol and, following addition of 20 units of the restriction enzyme PstI, the cleavage reaction was conducted at 37°C for 2 hours. Two $\mu$g of a tryptophan promoter-containing DNA fragment [PstI-SacI (T4 pol) fragment] of about 1,100 bp by the LGT method were recovered from the reaction mixture.

Further, separately, 10 $\mu$g of pGEL1 [Sekine et al., Proc. Natl. Acad. Sci. USA, 82, 4306 (1985)] were dissolved in 50 $\mu$l of Y-50 buffer, 20 units of the restriction enzyme PstI was added, and the cleavage reaction was conducted at 37°C for 2 hours. Then, NaCl was added to a concentration of 100 mM, 20 units of the restriction enzyme BamHI was added, and the cleavage reaction was conducted at 37°C for 2 hours. From the reaction mixture, there was recovered 2 $\mu$g of a replication origin-containing DNA fragment ( PstI-BamHI fragment) of 1,700 bp.

Then, 0.2 $\mu$g of the pcDIL-1-derived DpnI-BamHI fragment (about 1,000 bp) obtained in the above manner, 0.2 $\mu$g of the pTrS20-derived PstI-SacI (T4 pol) fragment (about 1,100 bp) obtained as described above and 0.3 $\mu$g of the above-mentioned pGEL1-derived PstI-BamHI fragment (about 1,700 bp) were dissolved in 20 $\mu$l of a buffer (hereinafter referred to as T4 ligase buffer) containing 20 mM Tris-HCl (pH 7.6), 10 mM $MgCl_2$, 10 mM dithiothreitol and 1 mM ATP and, after further addition of 2 units of T4 DNA ligase, the reaction was conducted at 4°C for 18 hours.

The thus-obtained recombinant plasmid DNA was used to transform Escherichia coli KM430 by the method of Cohen et al. [S.N. Cohen et al., Proc. Natl. Acad. Sci. USA, 69, 2110 (1972)], and $Ap^r$ colonies were obtained. The plasmid DNA was separated and purified from one of the transformants by a known method [H.C. Birnboim et al., Nucleic Acids Res., 7, 1513 (1979); hereinafter this method was always used for separation and purification of plasmid DNAs]. THe plasmid DNA was structurally analyzed by cleaving it with BamHI, PvuII, HindIII, EcoRI and other restriction enzymes. As a result, it was confirmed that the desired plasmid had been obtained. This recombinant plasmid was named pLIC12.

## EXAMPLE 2

Purification of IL-1

The Escherichia coli strain transformed with pLIC12 was grown in M9-casamino acids medium at 30°C for 15 hours. A 750-ml portion of the culture was centrifuged (5,000 × g, 10 minutes), and the cells collected were suspended in 200 ml of phosphate-containing physiological saline [composition: 0.8% NaCl, 0.02% KCl, 0.115% $Na_2HPO_4$, 0.2% $KH_2PO_4$] (hereinafter referred to as PBS) and disrupted by ultrasonication. Then, 200 ml of a supernatant was collected by centrifugation (5,000 × g, 10 minutes), and 55.4 g of ammonium sulfate was added to make a 45% saturated solution. Centrifugation (5,000 × g, 10 minutes) gave 200 ml of a supernatant. To this supernatant were added 19.8 g of ammonium sulfate, and the resultant precipitate was collected by centrifugation (5,000 × g, 10 minutes), dissolved in 20 ml of PBS and dialyzed against PBS to give 45 ml of a roughly purified IL-1 solution.

Then, 3 ml of this IL-1 solution was concentrated four times with Centricon 10 (Amicon), and 50 µl of the concentrate was purified by high-performance liquid chromatography (Toyo Soda TSK gel G3000 SW) to give 2 mg of highly purified IL-1.

EXAMPLE 3

Polypeptides showing IL-1 activity

A-4, A-3, V8-7, A-10 and V8-11

A 100 µg portion of the highly purified (at least 97% pure) IL-1 was dissolved in 10 mM Tris-4 M urea (pH 9.1) to make the whole volume 100 µl, 2 µg of API (Wako Pure Chemical Industries) was added, and the degradation reaction was conducted at 37°C for 5 hours. The reaction phase was subjected to reversed-phase high-performance liquid chromatography (Yamamura Kagaku's YMC AM312 ODS column). Elution on a gradient from 100% solution A (0.1% trifluoroacetic acid) to a mixture of 20% solution A and 80% solution B (0.1% trifluoroacetic acid-90% acetonitrile), in combination with assaying eluate fractions for IL-1 activity, gave 2 µg each of the polypeptides of Formula A-4, Formula A-3 and Formula A-10 (hereinafter referred to as A-4, A-3 and A-10, respectively, for short) in the order of elution.

Similarly, 100 µg of the purified IL-1 was dissolved in 100 mM Tris (pH 7.8), 2.5 µg of V8 protease (Miles Laboratories) was added, and the cleavage reaction was conducted at 37°C for 22 hours. The reaction mixture was subjected to reversed-phase high-performance liquid chromatography in the same manner as above and eluate fractions were assayed for IL-1 activity. In the order of elution, the polypeptides of Formula V8-7 and Formula V8-11 (hereinafter referred to as V8-7 and V8-11 for short) were obtained each in an amount of 2 µg.

The IL-1, A-4, A-3, V8-7, A-10 and V8-11 all showed IL-1 activity.

EXAMPLE 4

IL-1 activity assay

Activity evaluation was performed essentially by the method of Kasahara et al. [Rinsho Kensa (Journal of Medical Technology), 28 (3), 328 (1984)]. Thus, thymocytes from C3H/He mice of 4-6 weeks of age were suspended in RPMI 1640 medium (Nissui Pharmaceutical) supplemented with 10% fetal calf serum (FCS) (Gibco) and 0.5-1.0 µg/ml concanavalin A (ConA) in a concentration of $1 \times 10^7$ to $2 \times 10^7$ cells/ml. The thymocyte suspension was distributed in 0.1-ml portions into the wells of a 96-well microplate, together with appro priate dilutions of the polypeptide samples according to the invention, which were distributed in 0.1-ml portions, and the thymocytes were incubated at 37°C for 48 hours in air containing 5% carbon dioxide. Each well was pulse-labeled with 0.25 µCi of $^3H$-thymidine, and cells were recovered on the third day. The uptake of $^3H$-thymidine was determined by the subsequent liquid scintillation counting. One unit (standard unit) of activity is defined as the ability to induce 50% of the maximum $^3H$-thymidine uptake by thymocytes. The interleukin-1 activity values for the IL-1, A-4, A-3, V8-7, A-10 and V8-11 obtained in Examples 2 and 3 are shown in Table 2.

Table 2

| Peptide | Interleukin-1 activity, U/mg |
|---|---|
| IL-1 | $5 \times 10^6$ |
| A-4 | $10^8$ |
| A-3 | $10^8$ |
| V8-7 | $5 \times 10^7$ |
| A-10 | $3 \times 10^7$ |
| V8-11 | $7 \times 10^6$ |

EXAMPLE 5

For the purpose of evaluating the peptides obtained in Examples 2 and 3 for pyrogenicity, each peptide was administered intravenously to rabbits (3 animals per group) at the dose indicated in Table 3 and, at 1 hour after each administration, the body temperature rise was measured. In test run (a) alone using a substantial quantity of IL-1, a pyrogenically positive result was obtained while, in other runs, the results were negative; compare the result of test run (b) using one-tenth amount as test run (a). When compared in terms of the number of molecules, the dose of IL-1 in (a) and the doses of the peptides in (c), (e), (f), (g) and (h) were equivalent to one another. It was therefor established that these peptides show IL-1 activity with no, decreased or significantly decreased pyrogenicity (Table 3).

Table 3

| Test No. | Peptide | Dose | Body temperature rise (°C) | Judgment |
|---|---|---|---|---|
| (a) | IL-1 | 780 ng/kg | 0.60, 0.74, 0.16 | (+) |
| (b) | IL-1 | 78 ng/kg | 0.15, 0.04, 0.52 | (-) |
| (c) | A-4 | 32 ng/kg | 0.50, 0.27, 0.06 | (-) |
| (d) | A-4 | 3.2 ng/kg | 0.02, 0.43, 0.06 | (-) |
| (e) | A-3 | 42 ng/kg | 0.45, 0.08, 0.26 | (-) |
| (f) | V8-7 | 64 ng/kg | 0.15, 0.38, 0.04 | (-) |
| (g) | A-10 | 112 ng/kg | 0.06, 0.16, 0.42 | (-) |
| (h) | V8-11 | 309 ng/kg | 0.25, 0.60, 0.54 | (±) |

EXAMPLE 6

Neutrophilic phagocytosis-promoting activity

IL-1 and A-4 were evaluated for their neutrophilic phagocytosis-promoting activity by the method of Stossel et al. [T.P. Stossel, et al., J. Exp. Med., 137, 690 (1973)], as follows:

$2 \times 10^7$ neutrophils were suspended in phosphate-containing Krebs-Ringer solution (KRP) and incubated for 30 minutes together with an appropriate dilution of the sample. To this was added Oil Red O (Nakarai Chemicals), an opsonin-bound dye, and the dye was allowed to be taken up by neutrophils. The portion of Oil Red O taken up destroyed cells. The cells destroyed were extracted with chloroform and the extract was measured for the absorbance at 525 nm. The results thus obtained are shown in Table 4.

Table 4

| Sample | Absorbance | Judgment |
|---|---|---|
| Control | 0.1580 ± 0.0128 | |
| IL-1 100 ng/ml | 0.1821 ± 0.0263 | (+) |
| A-4 100 ng/ml | 0.1958 ± 0.0397 | (+) |

EXAMPLE 7

Chemical synthesis of A-4 peptide

The peptide synthesis was performed using an Applied Biosystems model 430A automatic peptide synthesizer and reagents and solvents purchased from Applied Biosystems in accordance with a standard operational program for the synthesizer. Thus, 0.88 g of a polystyrene solid phase carrier with α-t-butyloxycarbonyl-ε-chlorobenzyloxycarbonyllysine (0.5 millimole) bound thereto was placed in the reactor of the automatic synthesizer, and the α-amino group of lysine on the solid carrier was rendered free by elimination of the t-butyloxycarbonyl group with 50% trifluoroacetic acid-methylene chloride. Thereto was added a reaction mixture containing an active ester of asparagine (formed from 2 equivalents of α-t-butyloxycarbonylasparagine, 1 equivalent of dicyclohexylcarbodiimide and 1 equivalent of N-hydroxybenzotriazole), and the condensation reaction was conducted. These steps were conducted automatically according to the program, and asparaginyllysine (Asn-Lys) was synthesized on the solid phase carrier. Thereafter, t-butyloxycarbonyl group elimination with trifluoroacetic acid and amino acid condensation using dicyclohexylcarbodiimide as the activating agent were automatically repeated until Arg-Phe-Val-Phe-Asn-Lys was synthesized on the solid phase carrier.

After completion of the synthesis, the solid carrier weighed 1.20 g. A 500 mg portion of this carrier was suspended in 0.5 ml of anisole and the suspension was allowed to stand overnight at room temperature. Then, 5 ml of hydrogen fluoride was added, and the mixture was stirred for 1 hour with ice cooling. After removal of the hydrogen fluoride by distillation under reduced pressure, the residue was extracted with 30 ml of 1 M aqueous acetic acid solution. Thus was obtained, in a crude form, the peptide released from the solid phase carrier and deprotected entirely. The solution containing this peptide was washed with two 30-ml portion of ether and passed through a column packed with 6 ml of an ion exchange resin (Diaion SA-11). The column was washed with 20 ml of 1 M aqueous acetic acid solution. The eluates were collected and lyophilized. The residue was dissolved in 3 ml of 0.5 M aqueous acetic acid solution and the solution was applied to a Sephadex G-25 column ($\phi$ 2.4 $\times$ 84 cm). The column was eluted with 0.5 M aqueous acetic acid solution, and eluates were fractionated in 7-ml portions. Fractions Nos. 33-40 were combined and concentrated to give 211 mg of the peptide in an almost pure form. A portion of the peptide was further purified by high-performance liquid chromatography (HPLC) using a reversed-phase column (Yamamura Kagaku's YMC-ODS, $\phi$ 4.6 $\times$ 250 mm). Elution of the peptide in HPLC was performed by the linear concentration gradient method using 15% to 60% aqueous acetonitrile solutions containing 0.1%

trifluoroacetic acid. Thus was obtained in the peptide in a pure form. The structure of the peptide was confirmed by mass spectrometry (Hitachi model 80B spectrometer), in which the (M + 1) peak was detected at the mass number 810 and peaks were detected at 681, 567, 420 and 321, corresponding to successive amino acid elimination from the carboxyl terminus.

## REFERENCE EXAMPLE 1

### Isolation of human IL-1 cDNA-carrying plasmid pcDIL-1

(1) Preparation of poly(A) RNA from human macrophages

Normal human macrophages were separated by the method of Auron et al. [Philip E. Auron et al., Proc. Natl. Acad. Sci. USA, 81, 7907 (1984)] and activated with *Escherichia coli*-derived lipopolysaccharide. *Escherichia coli* lipopolysaccharide (Difco) was added to 5 × $10^8$ macrophages to a concentration of 300 ng/ml. After 4 hours of incubation, the culture supernatant was removed, and the cells were solubilized with 50 ml of a solution containing 5 M guanidinethiocyanate, 10 mM EDTA, 50 mM Tris-HCl (pH 7) and 8% (v/v) 2-mercaptoethanol. This solubilization product was transferred to a centrifuge tube, 350 ml of 4 M LiCl solution was added, and the mixture was stirred and then allowed to stand at 4°C for 20 hours. RNA was recovered as a sediment by centrifugation on a Hitachi RPR10 rotor at 6,000 rpm for 60 minutes. The sediment RNA was suspended in 200 ml of a solution containing 4 M urea and 2 M LiCl, and the RNA was again recovered as a sediment by centrifugation on a Hitachi RPR10 rotor at 6,000 rpm for 60 minutes. The RNA sediment was dissovled in 15 ml of a solution containing 0.1% sodium lauryl sulfate, 1 mM EDTA and 10 mM Tris-HCl (pH 7.5), then extracted with phenol-chloroform and recovered by precipitation with ethanol. About 2.0 mg of the thus-obtained RNA was dissolved in 1 ml of a solution containing 10 mM Tris-HCl (pH 8.0) and 1 mM EDTA. After incubation at 65°C for about 5 minutes, 0.1 ml of 5 M NaCl was added. The mixture was subjected to chromatography (column volume 0.5 ml) on an oligo(dT)-cellulose column (P-L Biochemicals). Poly(A)-containing mRNA thus adsorbed was eluted with a solution containing 10 mM Tris-HCl (pH 8.0) and 1 mM EDTA. Thus was obtained about 50 µg of poly(A)-containing mRNA.

(2) Synthesis of cDNA

Synthesis of IL-1 cDNA and construction of a recombinant plasmid with the cDNA inserted therein were performed using the Okayama-Berg method [Mol. Cell. Biol., 2, 161 (1982)]. The processes therefor are outlines in Fig. 2.

To 300 µl of a solution containing 10 mM Tris-HCl (pH 7.5), 6 mM $MgCl_2$ and 10 mM NaCl was added 400 µg of pCDV1 [Okayama & Berg, Mol. Cell. Biol., 3, 280 (1983)], then 500 units of *Kpn* I was further added, and the reaction was conducted at 37°C for 6 hours, whereby the plasmid was cleaved at the *KpnI* site. The DNA was recovered by phenol-chloroform extraction followed by precipitation with ethanol. About 200 µg of the *KpnI*-cleaved DNA was added to 200 µl of a solution prepared by supplementing a buffer (hereinafter referred to as TdT buffer) containing 40 mM sodium cacodylate, 30 mM Tris-HCl (pH 6.8), 1 mM $CaCl_2$ and 0.1 mM dithiothreitol (hereinafter abbreviated as DTT) with dTTP to a concentration of 0.25 mM. After further addition of 81 units of terminal deoxynucleotidyl transferase (hereinafter abbreviated as TdT) (P-L Biochemicals), the reaction was conducted at 37°C for 11 minutes, whereby a poly(dT) chain comprising about 67 dT units was joined to pCDV1 at each 3′ end thereof resulting from *KpnI* cleavage. About 100 µg of pCDV1 DNA with poly(dT) chains joined thereto was recovered from the solution by extraction with phenol-chloroform and precipitation with ethanol. The DNA was added to 150 µl of a buffer consisting of 10 mM Tris-HCl (pH 7.5), 6 mM $MgCl_2$ and 100 mM NaCl and, after further addition of 360 units of *EcoRI*, the reaction was conducted at 37°C for 2 hours. The reaction mixture was treated by the LGT method, and a DNA fragment of about 3.1 kb was recovered. Thus was obtained about 60 µg of pCDV1 with a poly(dT) chain joined thereto. The DNA was dissolved in 500 µl of a solution consisting of 10 mM Tris-HCl (pH 8.0) and 1 mM EDTA. The solution was incubated at 65°C for 5 minutes and then ice-cooled, and 50 µl of 5 M NaCl was added. The mixture was chromatographed on an oligo(dA)-cellulose column (Collabora tive Research). DNAs having a sufficiently long poly(dT) chain were adsorbed on the column. Elution with a solution consisting of 10 mM Tris-HCl (pH 8.0) and 1 mM EDTA gave 27 µg of pCDV1 with a poly(dT) chain joined thereto (hereinafter referred to as vector primer).

Then, a linker DNA was prepared.

About 14 µg of pL1 (Okayama & Berg, Mol. Cell. Biol., 3 , 280 (1983)] was added to 200 µl of a buffer consisting of 10 mM Tris-HCl (pH 7.5), 6 mM $MgCl_2$ and 50 mM NaCl and, after further addition of 50 units of PstI, the reaction was conducted at 37°C for 4 hours, whereby the pL1 DNA was cleaved at the PstI site therein. The reaction mixture was extracted with phenol-chloroform, and about 13 µg of the PstI-cleaved pL1 DNA was recovered by precipitation with ethanol. The DNA (about 13 µg) was added to 50 µl of TdT buffer containing dGTP in a final concentration of 0.25 mM and, after further addition of 54 units of TdT (P-L Biochemicals), the mixture was incubated at 37°C for 13 minutes, whereby a (dG) chain of about 14 (dG)s was joined to each 3' end resulting from PstI cleavage. The resultant DNA was recovered by extraction with phenol-chloroform followed by precipitation with ethanol. The thus obtained DNA was added to 100 µl of a buffer consisting of 10 mM Tris-HCl (pH 7.5), 6 mM $MgCl_2$ and 60 mM NaCl and, after further addition of 80 units of HindIII, the mixture was incubated at 37°C for 3 hours, whereby the pL1 DNA was cleaved at the HindIII site therein. The reaction mixture was fractionated by agarose gel electrophoresis, and a DNA fragment of about 0.5 kb was recovered by the DEAE paper method [Dretzen et al., Anal. Biochem., 112, 295 (1981)]. A linker DNA (hereinafter referred to as linker DNA for short) with an oligo(dG) chain joined thereto was obtained.

About 3 µg of the poly(A)-RNA and about 1.4 µg of the vector primer, each prepared as described above, were dissolved in 22.3 µl of a solution consisting of 50 mM Tris-HCl (pH 8.3), 8 mM $MgCl_2$, 30 mM KCl, 0.3 mM DTT and 2 mM dNTP (dATP, dTTP, dGTP and dCTP, each 2 mM) and containing 10 units of ribonuclease inhibitor (P-L Biochemicals) and, after addition of 10 units of reverse transcriptase (Seikagaku Kogyo), the mixture was incubated at 41°C for 90 minutes, whereby a DNA complementary to the mRNA was synthesized. The reaction mixture was extracted with phenol-chloroform, and the vector primer with an RNA-DNA double-stranded chain joined thereto was recovered by precipitation with ethanol. The DNA obtained was dissolved in 20 µl of TdT buffer containing 66 µM dCTP and 0.2 µg of poly(A) and, after addition of 14 units of TdT (P-L Biochemicals), the mixture was incubated at 37°C for 2 minutes, whereby a (dC) chain of 20 (dC)s was joined to the 3' end of cDNA. The reaction mixture was extracted with phenol-chloroform, and the cDNA-vector primer DNA with a (dC) chain joined thereto was recovered by precipitation with ethanol. The DNA was dissolved in 400 µl of a solution consisting of 10 mM Tris-HCl (pH 7.5), 6 mM $MgCl_2$ and 60 mM NaCl and, after addition of 20 units of HindIII, the solution was incubated at 37°C for 2 hours, whereby cleavage occurred at the HindIII site. Extraction of the reaction mixture with phenol-chloroform and precipitation with ethanol gave 0.5 picomole of a (dC) chain-joined cDNA-vector primer DNA. A 0.2-picomole portion of the DNA and 0.4 picomole of the above-mentioned linker DNA were dissolved in 100 µl of a solution consisting of 10 mM Tris-HCl (pH 7.5), 0.1 M NaCl and 1 mM EDTA, and the solution was incubated at 65°C for 10 minutes, then at 42°C for 25 minutes and further at 0°C for 30 minutes. The reaction mixture was adjusted to a total volume of 1,000 µl and to a composition: 20 mM Tris-HCl (pH 7.5), 4 mM $MgCl_2$, 10 mM $(NH_4)_2SO_4$, 0.1 M KCl and 0.1 mM β-NAD. To the reaction mixture was added 25 units of Escherichia coli DNA ligase (New England Bio-Labs), and incubation was performed at 11°C for 18 hours. The reaction mixture was supplemented with dNTPs each to a concentration of 40 µM and with β-NAD to 0.15 mM and, after addition of 10 units of Escherichia coli DNA ligase, 20 units of Escherichia coli DNA polymerase I (P-L Biochemicals) and 10 units of Escherichia coli rebonuclease H (P-L Biochemicals), incubation was conducted at 12°C for 1 hour and then at 25°C for 1 hour.

The above reactions resulted in cyclization of the cDNA-containing recombinant DNA and substitution of the RNA portion of the RNA-DNA double strand with the corresponding DNA. A recombinant plasmid in the form of a completely double-stranded DNA was thus formed.

(3) Selection of human IL-1 cDNA-containing recombinant DNA

Escherichia coli C600 SF8 [Cameron, Proc. Natl. Acad. Sci. USA, 72, 3416 (1975)] was transformed with the recombinant plasmid obtained in (2), by the method of Scott et al. [K. Shigesada, Saibo Kogaku (Cell Technology), 2, 616 (1983)]. About 3,000 colonies thus obtained were fixed on nitrocellulose filters.

One strain was selected that strongly associated at 42°C with $^{32}$P-labeled 5'-CGAGCTTAGGTACTTCT-3', a 17-base synthetic DNA probe identical in base sequence with a part (on the N-terminal side) of the human IL-1 cDNA isolated by Auron et al. [Philip E. Auron et al., Proc. Natl. Acad. Sci. USA, 81, 7907 (1984)] [the Grunstein-Hogness method, Proc. Natl. Acad. Sci. USA, 72, 3961 (1975)]. The whole base sequence of the cDNA in the plasmid pcDIL-1 carried by said strain was determined by the dideoxy sequencing method [J. Messing et al., Gene, 19, 269 (1985)]. As a result, it was determined that the cDNA in pcDIL-1 codes for the human IL-1 shown in Table 1.

REFERENCE EXAMPLE 2

Construction of ATG vector pTrS20

Following the scheme shown in Fig. 3, an ATG vector, pTrS20, was constructed in which the distance between the SD sequence and the initiation codon ATG is equal to 14 bases and a SacI site is located immediately behind the ATG codon.

First, 3 µg of pKYP10 prepared as described in Japanese Patent Application (OPI) No. 110600/83 (the term "OPI" means "unexamined published application") was dissovled in 30 µl of Y-100 buffer and, after addition of 6 units each of the restriction enzymes BanIII and NruI (New England Bio-Labs), the cleavage reaction was conducted at 37°C for 3 hours. From the reaction mixture, there was recovered about 0.5 µg of a DNA fragment (BanIII-Nru I fragment) of about 3.8 kb by the LGT method.

Separately, for providing the initiation codon ATG downstream from Ptrp, the following DNA linker was synthesized by the phosphotriester method:

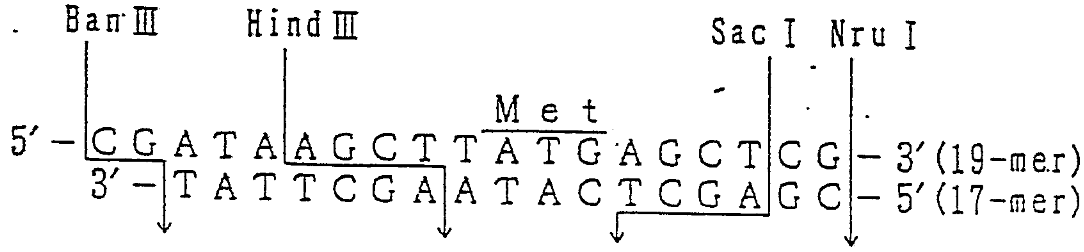


The 19-mer and 17-mer synthetic DNAs (10 picomoles each) were dissolved in 20 µl of a solution containing 50 mM Tris-HCl (pH 7.5), 10 mM $MgCl_2$, 5 mM dithiothreitol, 0.1 mM EDTA and 1 mM ATP, 3 units of T4 polynucleotide kinase (Takara Shuzo) was added, and the phosphorylation reaction was conducted at 37°C for 60 minutes.

Then, 0.1 µg of the above-mentioned pKYP10-derived Ban III-NruI fragment (about 3.8 kb) and about 0.5 picomoles of the above phosphorylated DNA linker were dissolved in 20 µl of T4 ligase buffer, 2 units of T4 DNA ligase was further added, and the ligation reaction was conducted at 4°C for 18 hours.

The thus-obtained recombinant plasmid mixture was used to transform Escherichia coli HB101 [Boliver et al., Gene, 2, 75 (1977)], and $Ap^r$ colonies were obtained. A plasmid DNA was recovered from the cultured cells derived from one of said colonies. The structure of the plasmid obtained was confirmed by cleavage with the restriction enzymes Eco RI, BanIII, HindIII, SacI and NruI followed by agarose gel electrophoresis. This plasmid was named pTrS20 (Fig. 3). Sequencing by the dideoxy method confirmed that the base sequence of pTrS20 in the neighborhood of th BanIII and HindIII sites is as follows:

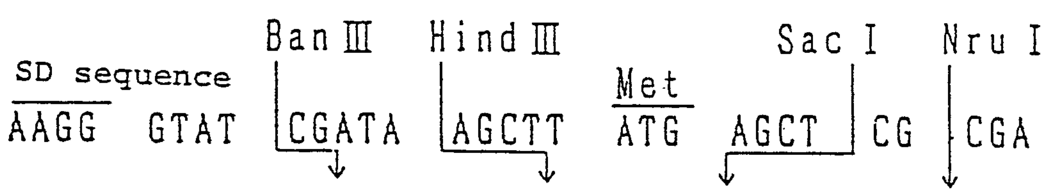


While the invention has been described in detail and with reference to specific embodiments thereof, it will be apparent to one skilled in the art that various changes and modifications can be made therein without departing from the spirit and scope thereof.

**Claims**

1. A polypeptide having interleukin-1 activity, substantially free of pyrogenicity in mammals and selected from the polypeptides having the following amino acid sequences:

(a) Arg-Phe-Val-Phe-Asn-Lys (Formula A-4),

(b) Lys-Arg-Phe-Val-Phe-Asn-Lys-Ile-Glu (Formula V8-7),

(c) Ser-Leu-Asn-Cys-Thr-Leu-Arg-Asp-Ser-Gln-Gln-Lys (Formula A-3),

(d) Asn-Tyr-Pro-Lys-Lys-Lys-Met-Glu-Lys-Arg-Phe-Val-Phe-Asn-Lys-Ile-Glu-Ile-Asn-Asn-Lys (Formula A-10), or

(e) Leu-Lys-Ala-Leu-His-Leu-Gln-Gly-Gln-Asp-Met-Glu-Gln-Gln-Val-Val-Phe-Ser-Met-Ser-Phe-Val-Gln-Gly-Glu-Glu-Ser-Asn-Asp-Lys-Ile-Pro-Val-Ala-Leu-Gly-Leu-Lys-Glu-Lys-Asn-Leu-Tyr-Leu-Ser-Cys-Val-Leu-Lys-Asp-Asp-Lys-Pro-Thr-Leu-Gln-Leu-Glu (Formula V8-11),

2. The polypeptides of Claim 1, obtainable by enzymatic treatment of human interleukin-1 or by chemical synthesis.

3. The polypeptides of Claim 2, wherein said enzyme is Achromobacter protease I or Staphylococcus aureus V8 protease.

4. DNA which codes for the polypeptides defined by the formulae A-4, V8-7, A-3, A-10 or V8-11 given in Claim 1.

5. A recombinant plasmid having the DNA of Claim 4 inserted therein.

6. A microorganism harboring the plasmid of Claim 5.

Fig. 1

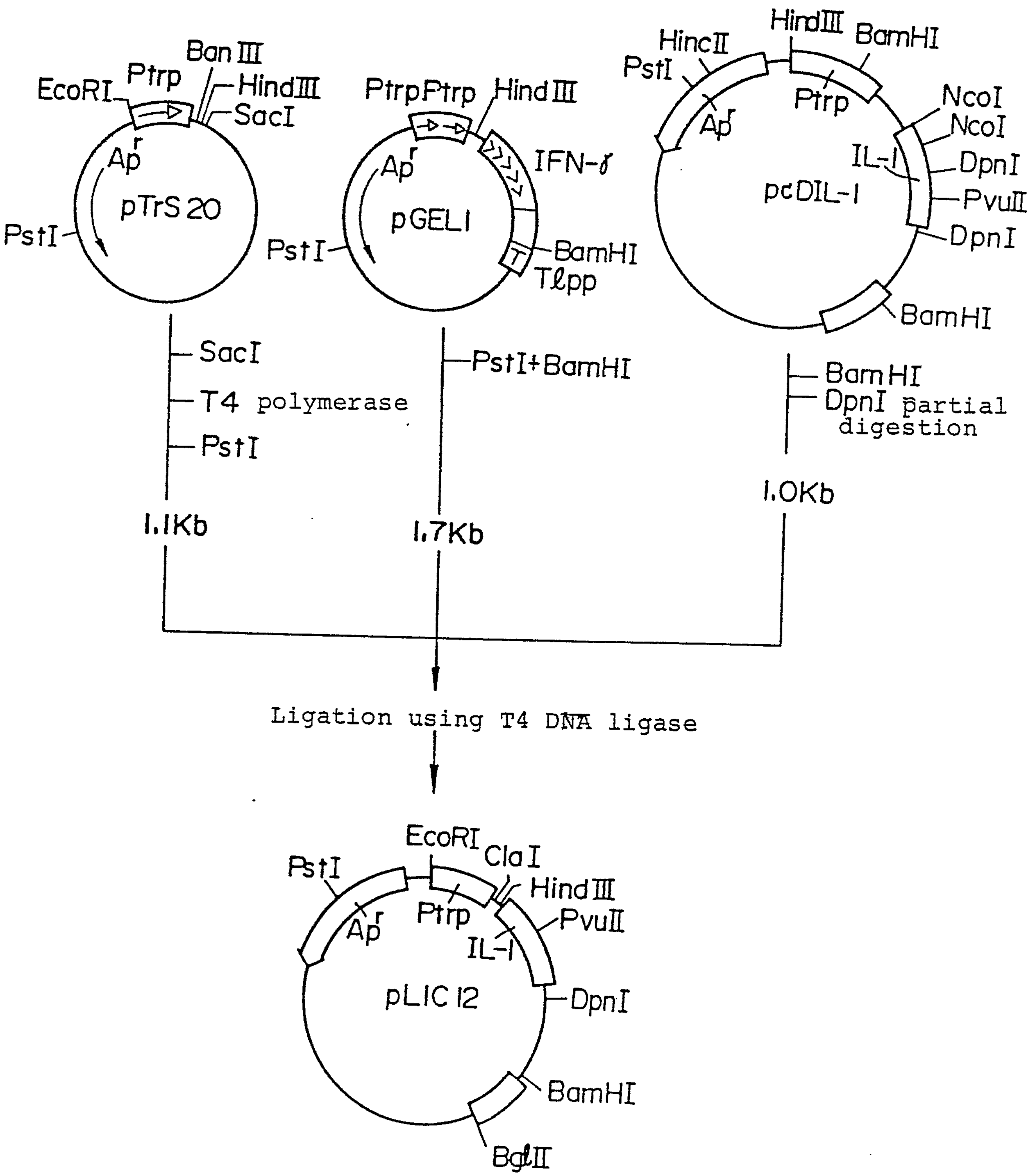

EcoRI
Ptrp
Ban III
HindIII
SacI
Ap^r
pTrS 20
PstI
PtrpPtrp
HindIII
IFN-γ
Ap^r
pGEL1
PstI
BamHI
Tℓpp
HincII
HindIII
BamHI
PstI
Ap^r
Ptrp
NcoI
NcoI
IL-1
DpnI
pcDIL-1
PvuII
DpnI
BamHI
SacI
T4 polymerase
PstI
1.1Kb
PstI+BamHI
1.7Kb
BamHI
DpnI partial digestion
1.0Kb
Ligation using T4 DNA ligase
EcoRI
ClaI
PstI
HindIII
PvuII
Ap^r
Ptrp
IL-1
pLIC 12
DpnI
BamHI
BglII

Fig. 2 (1)

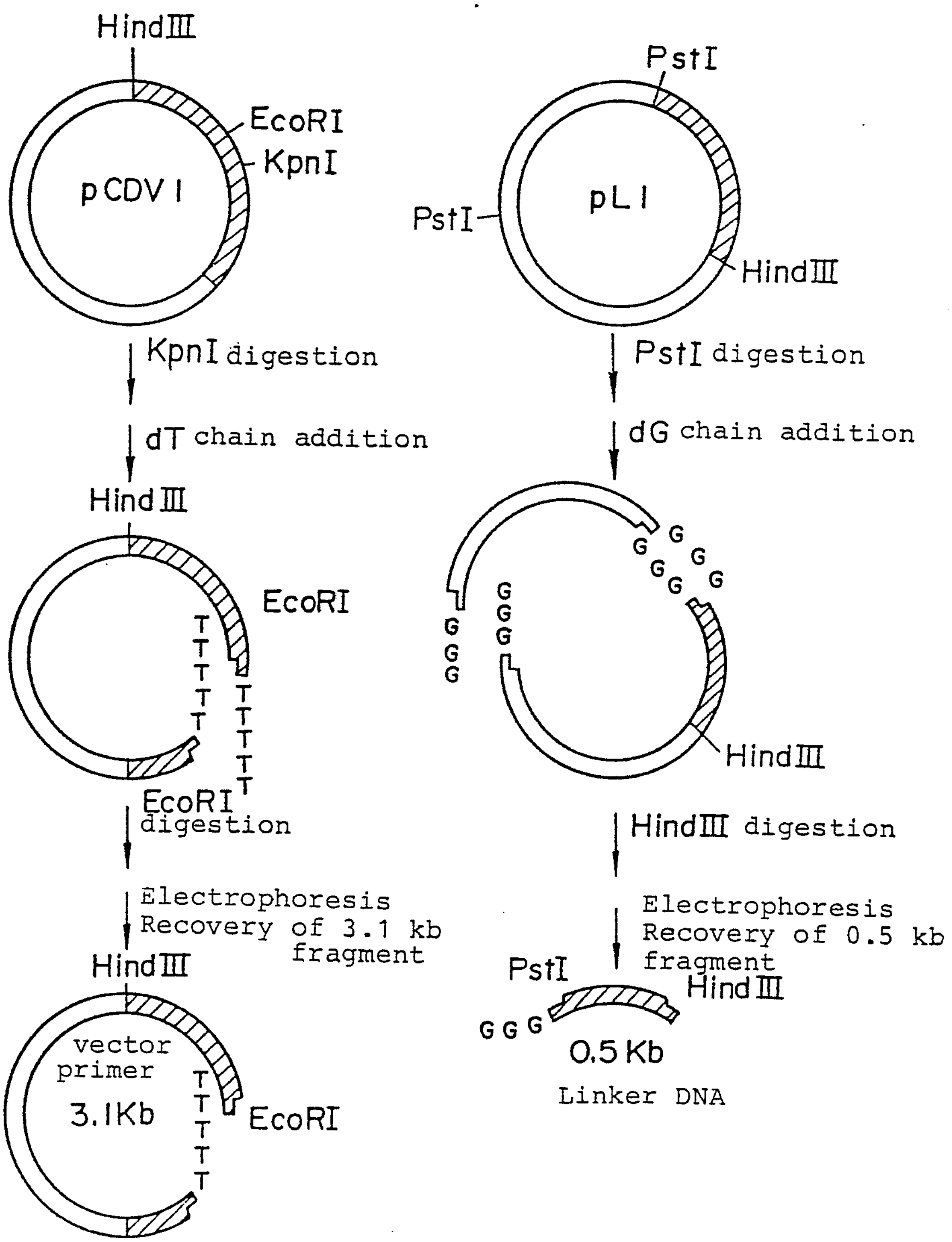
HindIII
EcoRI
KpnI
pCDV1
PstI
PstI
pL1
HindIII
KpnI digestion
dT chain addition
PstI digestion
dG chain addition
HindIII
EcoRI
HindIII
EcoRI digestion
Electrophoresis
Recovery of 3.1 kb fragment
HindIII digestion
Electrophoresis
Recovery of 0.5 kb fragment
HindIII
vector primer
3.1Kb
EcoRI
PstI
HindIII
GGG
0.5 Kb
Linker DNA

Fig. 2 (2)

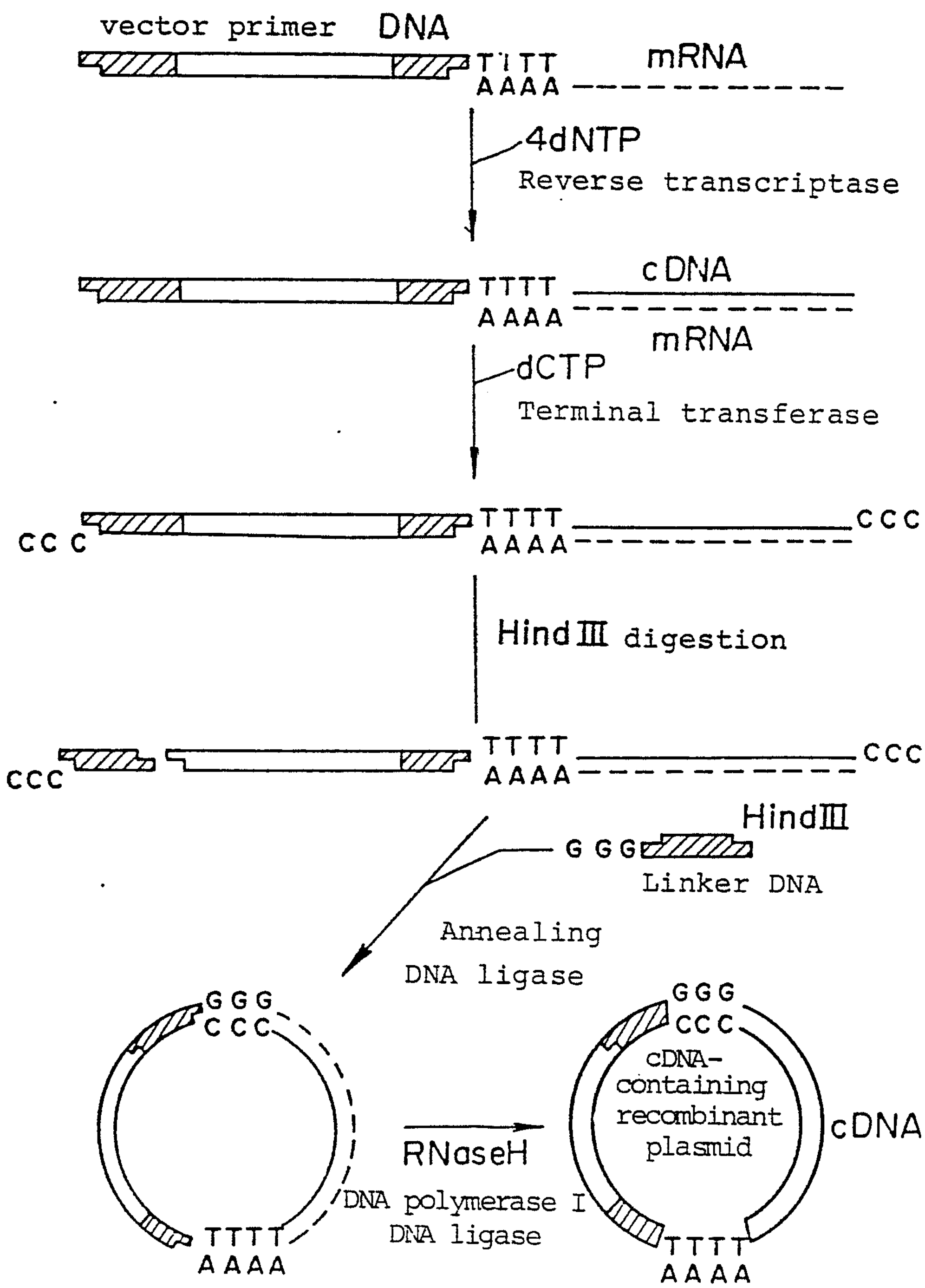
vector primer DNA
TTTT
AAAA
mRNA
4dNTP
Reverse transcriptase
TTTT
AAAA
cDNA
mRNA
dCTP
Terminal transferase
CCC
TTTT
AAAA
CCC
HindIII digestion
CCC
TTTT
AAAA
CCC
HindIII
GGG
Linker DNA
Annealing
DNA ligase
GGG
CCC
TTTT
AAAA
RNaseH
DNA polymerase I
DNA ligase
GGG
CCC
cDNA-containing recombinant plasmid
cDNA
TTTT
AAAA

Fig. 3

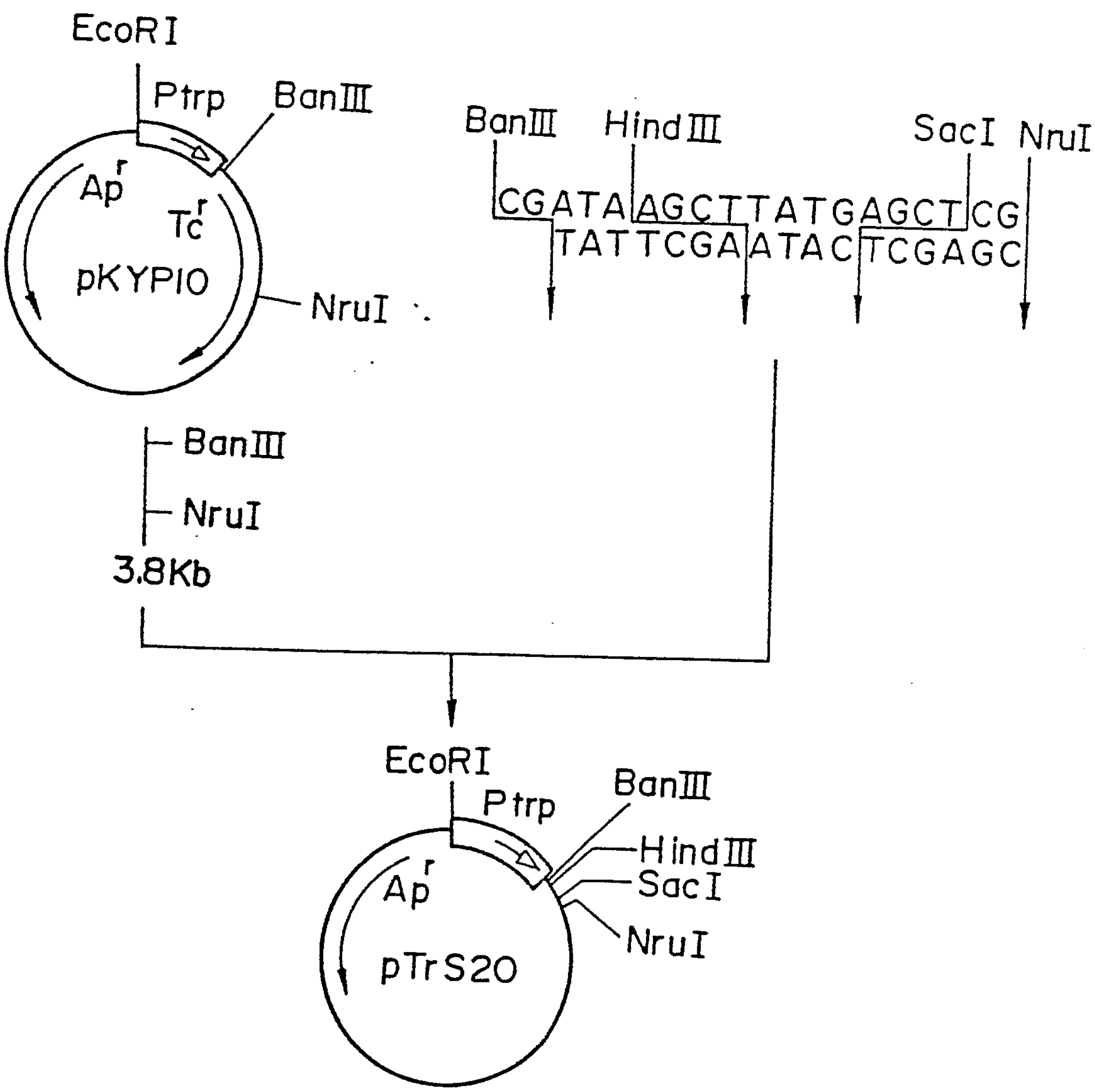
EcoRI
Ptrp
BanIII
Ap^r
Tc^r
pKYP10
NruI
BanIII
HindIII
SacI
NruI
CGATAAGCTTATGAGCTCG
TATTCGAATACTCGAGC
BanIII
NruI
3.8Kb
EcoRI
Ptrp
BanIII
HindIII
SacI
NruI
Ap^r
pTrS20